# EUROPEAN PATENT SPECIFICATION

(11) **EP 1 851 337 B1**
(45) Date of publication and mention of the grant of the patent: **10.10.2012**
(21) Application number: 06719536.2
(22) Date of filing: 25.01.2006
(51) Int. Cl.: C12Q 1/68, C12M 1/34, G02B 6/00, G02B 6/12, C12N 15/10

(54) **DNA PURIFICATION AND ANALYSIS ON NANOENGINEERED SURFACES**
DNS-REINIGUNG UND -ANALYSE AUF NANOTECHNISCH KONSTRUIERTEN OBERFLÄCHEN
PURIFICATION ET ANALYSE DE L'ADN SUR DES SURFACES FORMEES PAR NANO-INGENIERIE

(43) Date of publication of application: 07.11.2007
(73) Proprietor: Reed, Michael W., Seattle WA 98155 (US); Weigl, Bernhard H., Seattle WA 98103 (US); Bardell, Ronald L., St. Louis Park MN 55416 (US)
(72) Inventor: WEIGL, Bernhard, H., Seattle, WA 98103 (US); BARDELL, Ronald, L., St. Louis Park, MN 55416 (US); REED, Michael, W., Lake Forest Park, WA 98155 (US)
(74) Representative: Thomson, Neil David
(86) International application number: PCT/US2006/002708
(87) International publication number: WO 2006/081324

(56) References cited:
- WO-A-2005/066343
- US-A- 4 883 867
- US-A- 5 587 128
- US-A1- 2001 026 921
- US-A1- 2002 006 623
- US-A1- 2002 157 119
- US-A1- 2003 138 941
- US-A1- 2004 152 085
- US-B1- 6 377 721
- JOON-HO KIM ET AL: "A disposable DNA sample preparation microfluidic chip for nucleic acid probe assay" PROCEEDINGS OF THE IEEE 15TH. ANNUAL INTERNATIONAL CONFERENCE ON MICRO ELECTRO MECHANICAL SYSTEMS. MEMS 2002. LAS VEGAS, NV, JAN. 20 - 24, 2002; [IEEE INTERNATIONAL MICRO ELECTRO MECHANICAL SYSTEMS CONFERENCE], NEW YORK, NY : IEEE, US, vol. CONF. 15, 1 January 2002 (2002-01-01), pages 133-136, XP010577613 ISBN: 978-0-7803-7185-9

## Description

### BACKGROUND OF THE INVENTION

### 1. Field of the Invention

The present invention relates generally to the purification of DNA, and, in particular, the use of nanoengineered surfaces in the purification and analysis of DNA.

### 2. Description of the Prior Art

Technology to simplify the isolation, handling and measurement of genomic DNA is required for portable medical diagnostics or biodefense applications. The quality of nucleic acid isolated from human tissue is critical for reproducible, accurate and informative genetic analysis data. As the commercial importance of DNA isolation products has grown, there has been much work done in this area.

One technique in which much progress has been made is the use of microfluidic devices to perform genetic analysis. Many microarray assay formats are available, and they share the ability to allow simultaneous interrogation of a vast number of genetic targets from a single sample.

Sensitive solution phase assays for measuring concentration of double stranded DNA (dsDNA) have already been developed using fluorogenic minor groove binding molecules (MBs). When DNA is not present, the molecule can rotate freely in solution, and this is critical for its low fluorescent background. Upon binding to dsDNA, the compound assumes a rigid planar conformation in the hydrophobic minor groove that has increased fluorescence. By carefully engineering the attachment of fluorogenic MBs to surfaces, the quantity of DNA can be measured as it is transferred through a microfluidic device.

US 2001/026921, WO 2005/066343, US 2003/138941, US 5, 587,128, US 2004/ 152 085 and Joon-Ho Kim et al: Proceedings of the IEEE 15th Annual International Conference on Micro Electro Mechanical Systems. MEMS 2002. Las Vegas, NV, Jan. 20 - 24, 2002; [IEEE International Micro Electro Mechanical Systems Conference], New York, NY: Ivol. Conf. 15, 1 January 2002 (2002-01-01), pages 133-136; Boom et al., J. Cl. Microbiol., vol. 28, pages 495-503, 1990 and Vogelstein et al., PNAS. USA, vol. 76, pages 615-619, 1979 disclose use of modified surfaces for nucleic acid capture.

Therefore an object of the present invention to provide a microfluidic device that simultaneously isolates genomic DNA, measures the amount of purified DNA, and distributes standardized DNA solutions of specific concentration for downfield analysis is provided

Further a microfluidic device for DNA isolation and preparation in which the cost and complexity of the device is minimized is provided.

Described is a microfluidic device that can simultaneously capture double stranded DNA (dsDNA) from biological fields and measure the amount of DNA immobilized.

Further to synthesize fluorogenic minor groove binder agents (MBs) with electrophilic or nucleophilic linker groups and demonstrate increased fluorescence in the presence of dsDNA is described.

These and other objects of the present invention will be more readily apparent from the descriptions and drawings which follow.

### BRIEF DESCRIPTION OF THE DRAWINGS

FIG. 1 is a depiction of the structure of Hoechst 33258 bound to a synthetic 12- mer DNA duplex;
FIG. 2 shows the structure of reactive analogs of H33258 developed for attachment to linkers on DNA probes;
FIG. 3 is a depiction of the interaction of two fluids flowing within a microfluidic device;
FIG. 4 is a depiction of several amine-modified surfaces with attached fluorogenic minor groove binders (MBs);
FIG. 5 shows the synthesis of fluorogenic MBs with reactive groups;
FIG. 6 is a depiction of the activation of amine modified glass surfaces of a slide with cyanuric chloride in an organic solution;
FIG. 7 shows possible chamber designs for a microfluidic device suitable for use in the present invention;
FIG. 8 is a front view of a microfluidic card suitable for use in the present invention;
FIG. 9 is an exploded view of a microfluidic card with its layers separated;
FIGS. 10 A-E, taken together, depict a front view of the card of FIG. 9 showing the sequence of events of the card while in use;
FIG. 11 shows the synthesis of the fluorogenic Hoechst dye with an attached hexylamine linker;
FIG. 12 is a chart showing the fluorescence of the BB-NH² molecule vs. BB-OH excited at 360 nm.
FIG. 13 is a chart showing the fluorescence at 460 nm for four different bisbenzimide surfaces; and
FIG. 14 is a chart showing the amount of DNA bound versus the amount of DNA offered for glass and plastic cover slips.

### DESCRIPTION OF THE PREFERRED EMBODIMENTS

Sensitive solution phase assays for measuring the concentration of ds DNA have already been developed using fluorogenic minor groove binder (MB) agents (Hoechst 33258). When DNA is not present, the molecule can rotate freely in solution; this is critical for its low fluorescent background. Upon binding to dsDNA, the compound assumes a rigid planar conformation in the hydrophobic minor groove that has increased fluorescence. By carefully engineering the attachment of fluorogenic MBs to surfaces, the quantity of DNA can be measured as it is transferred through a microfluidic device. Conjugation chemistry is used to position the fluors on macromolecular structures designed to maximize conformational flexibility and access to target DNA.

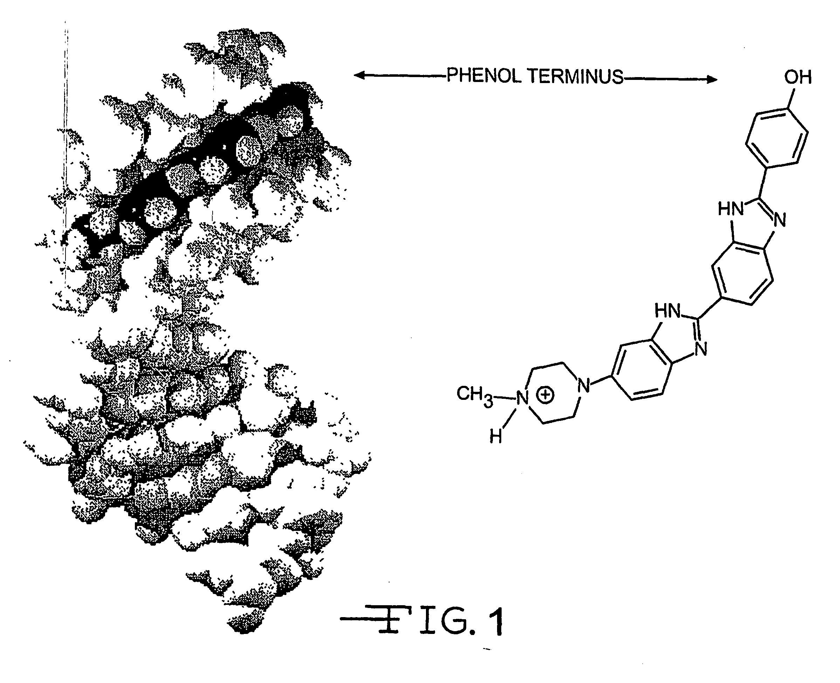

The Hoechst 33258 molecule has a polycyclic structure that can form a crescent shaped structure which is isohelical with the minor groove of B-form DNA. FIG. 1 shows the solution NMR structure of Hoechst 33258 bound to a synthetic 12 - mer DNA duplex (PDB structure ID number: 1QSX). The phenol terminus of the crescent shaped molecule is a convenient attachment point for attachment in conjugate groups. The structure in aqueous solution is flexible, and relatively non-fluorescent. Excitation of the unbound molecule with 356 nm light gives weak fluorescence at 492 nm. However, where bound to dsDNA, the benzimidazole rings become fixed in a planar conformation, and an extensive aromatic system develops that emits strong fluorescence at 458 nm. The molecule is shielded from water molecules in the minor groove, and this further increases fluorescence. These fluorogenic properties of H33258 have led to the development of sensitive assays for measuring dsDNA concentrations and, as a simple assay, for cell counting.

There has been much interest in the structure of H33258 bound to DNA. The MB is held in place by a combination of hydrophobic interactions, van der Waals forces, and hydrogen binding by the imidazole residues. The strong minor groove binding of H33258 may also provide a mechanism for direct isolation of dsDNA. Minor groove binding of MBs to adenine/thymine (A/T) rich sequences is preferred, and the DNA binding constants can be orders of magnitude larger than other small MW agents such as intercalators. Intercalating fluorgenic agents have also been extensively explored. An example of this class of DNA binding agents is ethidium bromide, which is commonly used in molecular biology to stain DNA in gels after electrophoresis. Methidium dyes have been attached to sepharose particles to provide a DNA isolation product. Intercalating dyes like methidium are not as strong binding as the minor groove binder class of dyes. Methedium was attached to sepharose particles using a cationic spermine linker, and this undoubtedly improved DNA binding. In addition, ethidium only shows a small increase in quantum yield upon binding to dsDNA. The fluorescent properties of the methidium-sepharose particles were not described.

The cyanine type intercalating dyes are much more fluorgenic than ethidium intercalators. Thiazole orange (TO) is an asymmetric cyanine dye composed of two bicyclic aromatic ring structures. When excited with 509 nm light, these two structures can rotate freely with respect to each other, and there is almost no fluorescence. However, in the presence of dsDNA, TO intercalates between base pairs and forces the ring systems to become planar. An extensive aromatic system forms that emits strong fluorescence at 533 nm. TO does not bind as tightly to dsDNA as the minor groove binding agents, but two TO molecules can be linked together to form strong binding bis-intercalators.

Sensitive DNA binding assays have been developed based on the fluorogenic properties of the cyanine type dyes. TO also shows up to 3000 fold increase in fluorescence in the presence of RNA. This property is interesting, and may be applicable to an RNA isolation device.

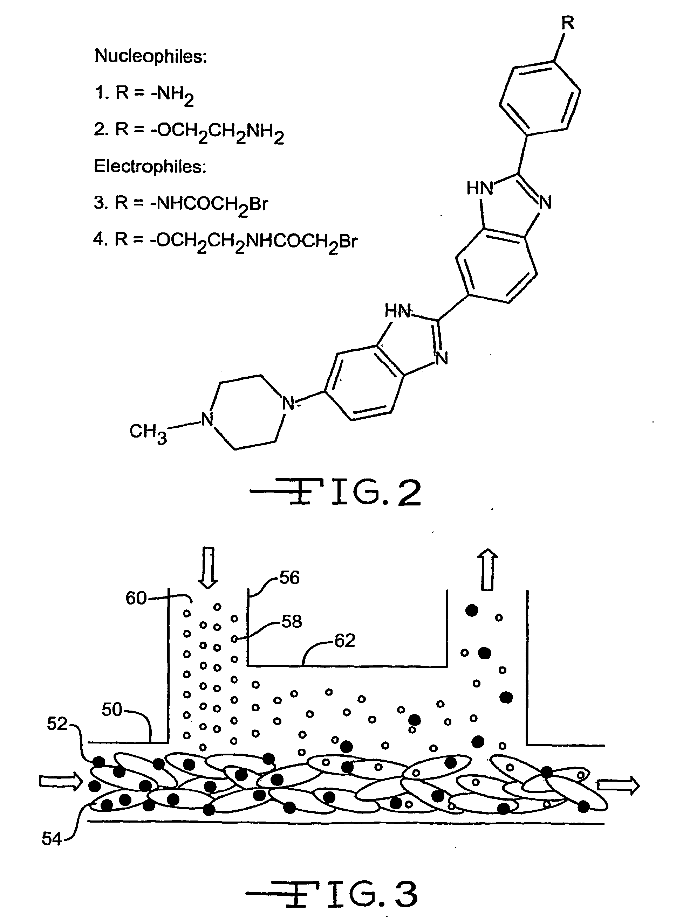

These fluorogenic compounds have been tethered to oligonucleotides to create DNA probes that can monitor the formation of dsDNA in hybridization assays. Several reactive derivatives of H33258 were developed for attachment to DNA probes. FIG.2 shows several reactive analogs of H33258 which were developed for attachment to linkers on DNA probes. For example, bromoacetyl analogs of H33258 were conjugated to sulfhydryl modified oligonucleotides. Sensitive DNA binding assays have been developed based on flurogenic properties of the cyanine type dyes. TO also shows up to 3000 fold increase in fluorescence in the presence of RNA. This property is interesting, and may be applicable to an RNA isolation device.

These fluorogenic compounds have been tethered to oligonucleotides to create DNA probes that can monitor the formation of dsDNA in hybridization assays. Several reactive derivatives of H33258 were developed for attachment to DNA probes. FIG. 2 shows several reactive analogs of H33258 which were developed for attachment to linkers on DNA probes. Hybridization to complimentary single strand DNA targets gave increased binding affinity and up to 23-fold increase in fluorescence upon binding. The improved binding efficiency (DNA melting temperature) was sequence specific. When A/T regions were located near the attachment point of ligand, TM dramatically increased, indicating the strong DNA binding effects of H33258. Similar synthetic chemistry may be used to prepare reactive analogs of these fluorogenic dyes and conjugate them to amine modified glass or plastic surfaces.

Cyanine dye conjugates of oligos also gave the expected hybridization triggered fluorescence upon addition of complementary strands of DNA. Fluorescence varied with the terminal sequence of the formed duplex. Binding of TO to DNA duplexes is known to be sequence specific. NHS ester analogs of cyanine dyes were reacted with amine modified oligonucleotides to give DNA probes that were used in fluorogenic "real-time" PCR assays. Up to 20-fold increase in fluorescence was observed with the oligonucleotide conjugates. DNA probe applications of both MGB and cyanine-type fluorogenic oligonucleotide conjugates were complicated by sequence specific binding of the dyes. Measurement of dsDNA binding should not be affected by sequence specificity of the binding sites, as the focus is heterogeneous populations of genomic fragments of DNA.

Nucleic acids have a very high affinity for glass surfaces in the presence of high concentrations of chaotropic salt solutions, and a number of glass based DNA isolation products are commercially available. These products are simple to use, but require multiple pipetting steps, Eppendorf tubes or 96-well plates, and access to equipment such as vortexers, and centrifuges. The operations take place in an open lab and require relatively large sample volumes to avoid problems with evaporation of solutions. DNA concentrations are usually measured by UV-vis spectrophotometers, and good technique is required for reproducible results. Nonetheless, use of glass surfaces for isolation of DNA from a variety of biological samples has been demonstrated.

An early application of DNA binding to glass was for isolation of electrophoretically purified DNA from agarose gels. The DNA binding capacity of flat soda lime or borosilicate glass surfaces were comparable: approximately 300 ng of DNA per 800 mm². 800 mm² is roughly the surface area of a chamber formed between a glass microscope slide and a 20x20 mm cover slip. Most macrofluidic DNA isolation applications use powdered glass from tubes or fibers, silicon dioxide, or the silica skeletons of diatoms, as these supports have a much higher surface area than flat surfaces. A microfluidics device can use lower binding flat glass surfaces since the volumes of solutions are smaller (no evaporation problems) and the amount of captured DNA required for PCR based genomic analysis is very small. Flat glass is available in a variety of compositions, sizes, and roughness, and can be further etched if more surface area is needed.

Solution phase analysis of mRNA or DNA can be accomplished during PCR using DNA oligonucleotide probes bearing fluorescent reporter groups at one terminus and a fluorescent quencher molecule at the other. In one format (the TaqMan assay), the probes are short single stranded oligos that are digested by the 3'-exonuclease activity of Taq polymerase. The other fluorogenic format (Molecular Beacon assay) uses hairpin shaped oligos with adjacent fluor and quencher molecules at the stem. The hairpins open in the presence of complementary DNA strands to give a fluorescent signal. Each of these fluorogenic formats can be used to monitor the progress of PCR, as each successful amplification cycle gives an increase in fluorescent signal. Both of these PCR based formats are ideal for microfluidics based platforms since the lower limit of assay size is only dictated by handling problems (drying of small volumes) and resolution of the available instruments. A popular (high-throughput) thermal cycling fluorimeter is the ABI7900HT which can analyze 384 well plates, with 10uL per well for each gene that is measured during PCR. 10 pg - 10 ng of genomic DNA per well is recommended, depending on efficiency of the PCR system, and the quality of the DNA.

Glass microscope slides are a common substrate for DNA microarrays. Many microarray assay formats are available, and share the ability to allow simultaneous interrogation of vast number of genetic targets from a single sample. Arrays of synthetic oligonucleotides have been synthesized directly from glass supports, or prepared by endpoint attachment of modified synthetic probes. Amine modified glass is a popular substrate for endpoint attachment. Gamma aminopropyl silane (GAPS) is a volatile reagent that can be used to functionalize glass surfaces and there are several manufacturers of GAPS slides. The amine coated surface of GAPS slides can be activated using bifunctional linkers to create an electrophilic surface which react with solutions of amine modified oligos. Variations of this conjugation chemistry may be used to attach fluorogenic DNA binding dyes.

The density of attachment of oligonucleotides is an important factor in hybridization performance. High densities of oligos can be achieved by direct synthesis from glass, but it has been shown that hybridization of labeled DNA targets actually dropped when the capture probes were too tightly packed. The hybridization performance also improved when the length of the linker between the glass attachment point and the oligonucleotide sequence was lengthened. Polyethylene glycol (PEG) linkers up to 80 atoms in length continued to improve hybridization signal. This shows the importance of long linkers between the solid support and the DNA probe in order to mimic the solution phase performance.

Attachment of DNA probes to glass surfaces via long polyethylene glycol linkers improved performance. Macromolecular PEG linkers with various functional groups are commercially available (Quanta, Biodesign, Powell, Ohio) and have been used primarily to attach to protein drugs to improve pharmacokinetic properties. Other macromolecular structures have r been used to improve performance of immobilized DNA probes. For example, biotin modified molecular beacons have been attached to avidin coated glass supports for use in sequence specific DNA analysis. The use of avidin or streptavidin as spacers to position the DNA away from the glass surface is likely important for the function of the probes. Another type of macromolecular linkers to consider are dendrimeric polymers. These commercially available (Aldrich Chemical Co., Milwaukee, WI) molecules are synthesized in alternating layers of amino groups and carboxylate groups depending on the "generation" of synthesis. The 3-dimensional structures of these molecules are well controlled, and the properties are well understood. Amine rich dendrimeric structures are analogous to the histone proteins that are critical for DNA packaging in cells. They have been used successfully for transfection of DNA sequences into cells for potential gene therapy applications. It is believed that dendrimeric linkers will provide excellent scaffolds for presenting immobilized MB molecules into DNA rich solutions. Biosensor films containing dendrimer linkers and fluorogenic DNA detecting molecules (Sytox 13, Molecular Probes) were used to measure levels of live bacteria on plastic surfaces. These materials did not contain a covalent link between the linker, fluorogenic dye or plastic surface, but were held together by hydrophobic forces.

DNA microarray technology provides miniaturized assays that benefit from enclosed microfluidics processing. For example, a micromachined chemical amplifier operated with continuous flow demonstrated a 20-cycle polymerase chain reaction (PCR) amplification of a 176-base pair fragment. PCR amplification of genomic DNA targets from white blood cells captured behind weirs in the flow chamber has been demonstrated from whole blood in silicon-glass microchips. A moderate density array method using DNA chips arrays was developed for diagnostic sequencing of PCR products to reduce their cost and complexity in use. Antibiotic resistant clinical isolates were visually detected within one hour after PCR amplification. Another application is the DNA sequence-based identification of toxic medicinal plants used in Traditional Chinese medicine. In 2001, Petrik reviewed the use of microarray devices originally developed for genomic projects for mass screening of blood donations for hepatitis C virus. An integrated system for PCR analysis was attained by combining dual Peltier thermoelectric elements with electrophoretic sizing and detection on a microchip. Using a DNA concentration injection scheme enabled detection of PCR products within as few as ten thermal cycles. A microfluidic cartridge was developed to prepare spores for PCR analysis by sonication, addition of PCR reagent to the disrupted spores, and insertion of the mixture in a PCR tube. The processing and detection of the spore DNA was completed within 20 minutes. An integrated microfluidic device was developed that combines stochastic PCR amplification of a single DNA template molecule followed by capillary electrophoretic analysis of the products. A histogram of the normalized peak areas from repetitive PCR analyses revealed quantization due to single viable template molecule copies in the reactor. A microfluidic chip for detecting RNA amplified by nucleic-acid-sequence-based amplification (NASBA) was developed. Samples of cryptosporidium parvum were detected and clearly distiguishable from controls without separating the amplified RNA from the NASBA mixture. An automated microfluidic system for nanoliter DNA analysis directly from cheek cells has been developed, including all the steps needed for DNA analysis: injection, mixing, lysis, PCR, separation, sizing, and detection. The possibility of further miniaturization of the system was established. An integrated microfluidic chip-based system has been developed for quality control testing of a recombinant, adenoviral, gene therapy product. The viral identity test sizes and quantitates the DNA fragments and requires 100-fold less sample than the agarose gel method.

It is desired to develop a DNA isolation device that will process a small volume of human blood (<20 µL) and deliver standardized genomic DNA samples for genetic analysis. A single use microfluidics card can be developed which may be inserted into a micropump driven instrument that controls fluid flow through various DNA processing chambers. The immobilized and quantitated DNA is either released in solution for immediate use, or stored for future analysis. The ability to measure the capture and release of immobilized DNA in the device by fluorescence will allow in-process control of this novel DNA processing system. The technology can be developed into a stand-alone product for general use in molecular biology research, or be incorporated into more sophisticated devices to simplify genetic analysis for biomedical applications. The standardized solutions of purified and denatured DNA that are isolated with the single well device developed can be distributed via microfluidic channels to various microwell plate formats for genetic analysis by real-time PCR or DNA microarrays. FIG. 3 is intended to display the micro-scale physics needed for development of miniaturized biomedical devices that can be used for isolation of genomic DNA from cells.

FIG. 3 shows the interaction between whole blood and another fluid flowing within the channels of a microfluidic device where isolation of DNA from whole blood is envisioned. It is envisioned that microscale physics used in the development of miniaturized biomedical devices can be used for isolation of genomic DNA from cells. Referring now to FIG. 3, a first stream 50 containing small particles 52 in whole blood 54, and a second stream 56 containing small particles 58 in a clear solution 60, are introduced into a common channel 62, where they form a laminar fluid diffusion interface (LFDI). Depending on their diffusion coefficient, particles 52, 58 start to diffuse across the LFDI, with the smaller particles 58 diffusing more quickly. The device can be used to extract small particles from whole blood, or to introduce a reagent into whole blood in a predictable continuous way.

A preliminary observation from studies of Hoechst dyes is also pertinent to this application. A droplet of Hoechst dye in water or buffer solutions shows increased fluorescence as the spot dries. This relates to the "hydration content" of the fluor environment and effects on fluorescent background. The fluor must be well hydrated for low background, and stresses the importance of hydrophilic surface coatings.

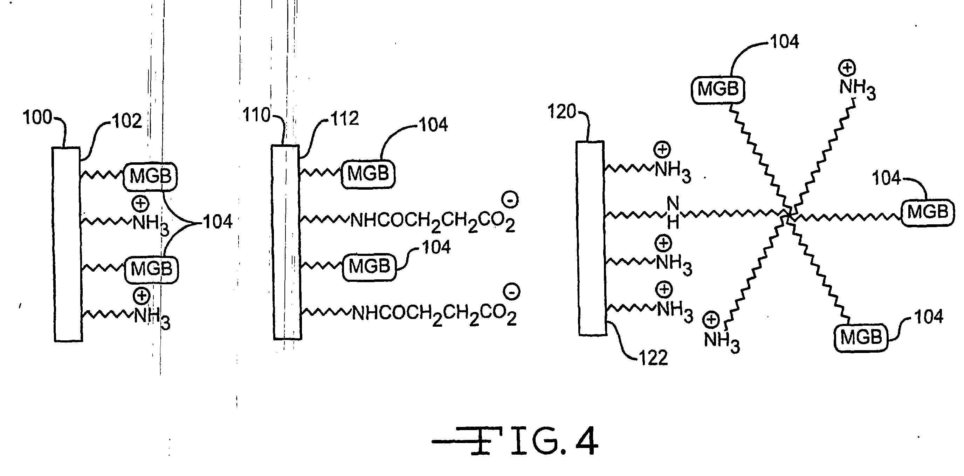

Initial focus will be synthesis and testing of fluorogenic solid supports for binding and measurement of dsDNA. Fluorogenic MBs with amine reactive functional groups will first be prepared using variations of known methods. These agents will be immobilized to amine modified glass or plastic, and fluorescence will be measured before and after treatment with DNA. Various linker structures will be evaluated with respect to their ability to measure fluorescence vs. DNA concentration. Fluorogenic surfaces with attached MBs will be engineered to provide low fluorescent background and good DNA binding. Illustrations of typical surfaces to be studied are shown in FIG. 4. Variables such as solid support (glass or plastic), linker chemistry, and capping groups will be explored. Stoichiometry (density of attachment) of fluorogenic MBs will be examined to determine the surface with the best dynamic range for measurement of DNA. DNA binding capacity will also be examined.

Referring now to FIG. 4, device 100 has an amine-modified surface 102 with attached minor groove binders (MB) 104. Unreacted amines can be left to provide a cationic surface. Device 110 shows that unreacted amines can be capped by succinylation to provide an anionic surface 112. Device 120 shows that MBs 104 can be extended into solution by first attaching a polymeric amine containing linker (starburst dedrimers, diamino-PEG, Poly-L-lysine) to surface 122 and further reacting with MB derivatives. Concurrent with the development of the required fluorogenic surfaces, a microfluidics device for DNA isolation is envisioned. Various size DNA samples will be obtained (Sigma), and efficiency of capture will be determined by fluorescence measurement in both solid phase and solution phase using the same fluorogenic compounds. Release of the DNA from the device will be monitored to ensure that DNA purity and recovery are good. Conditions for the capture and release of DNA will be measured on a fluorescent plate reader. After the DNA binding capacity and washing experiments are executed, a microfluidic device can be constructed. After the fluorogenic supports are developed, the DNA capture and measurement functions of the device can be combined.

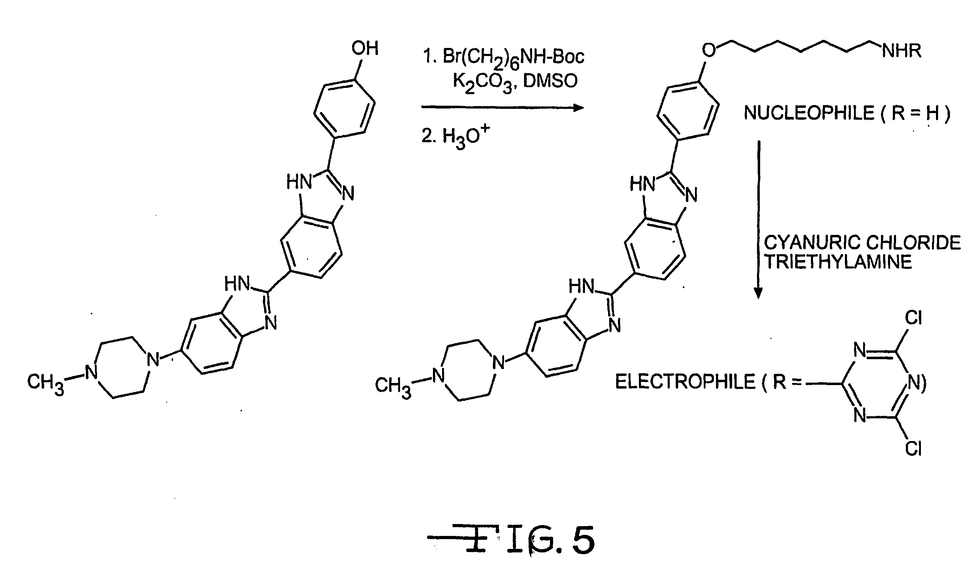

Since a variety of amine modified solid supports are available, derivatives can be prepared with active electrophilic functional groups such as iodoacetate groups, cyanuric chloride groups, NHS ester groups, and other amine reactive moieties. There are two synthetic approaches that have been shown to give H33258 analogs with desired electrophilic or nucleophilic "handles" that can react with complementary functional groups on the solid support. The most direct route is from the fully assembled heterocyclic ring system of H33258. Reaction of an bromoalkyl derivative of PEG has been shown to react with H33258 under basic conditions to give the aryl ether in high yield. H33258 is available from Aldrich Chemical Co. (500 mg = $220.80). The t-butyloxycarbonyl protected hexylamine linker is easily synthesized, and a tosylate derivative has been made. The bromo derivative should be easily accessible and reacted with H33258, as is shown in FIG. 5. Referring now to FIG. 5, Hoechst 33258 is treated with an alkylating Boc derivative. The nucleophilic aminoalkyl group (R=H) can be immobilized directly to the electrophilic surfaces. Alternatively, the amino group can be converted to an electrophilic group such as the cyanuric chloride derivative, as shown in FIG. 5, for immobilization to nucleophilic surfaces. The Boc is removed by treatment with dilute acid, and then neutralized with base to give the primary hexylamine group (R = H). The nucleophilic primary amine can react selectively with electrophiles in solution with little competing alkylation by the tertiary amine. This chemistry should be applicable to modification of solid surfaces that are coated with electrophilic functional groups. Alternatively, the H33258 hexylamine analog can be reacted with a bifunctional linker that leaves an electrophilic residue. Cyanuric chloride behaves like a bifunctional linker to join to amine containing functional groups. The remaining third chloro group is unreactive after the two amino groups are linked. This conjugation chemistry can be utilized later for synthesis of modified surfaces. Another alternative is to bind the cationic H33258 to anionic surfaces, which as that formed by carboxylic acid groups. These surfaces may be suitably stable and flexible to allow the desired fluorogenic response with DNA.

Like most solid phase chemistry, large excess of reagent (with respect to available surface groups) can be easily achieved and the surfaces are easily washed to remove excess reagent. Therefore, coupling efficiencies can be quite high (dense packing can be achieved). Surface area of glass and plastic substrates, immobilization chemistry, linker type and density of "linkable" fluorogenic compounds (fluors) will be optimized. Commercially available dendrimeric linkers, poly-L-lysine, and polyethylene glycol diamines should position the fluors away from the surface and toward dsDNA in solution. The goal is to maximize conformational flexibility of the immobilized fluors (to minimize background) and maximize accessibility to dsDNA (to maximize DNA binding and kinetics).

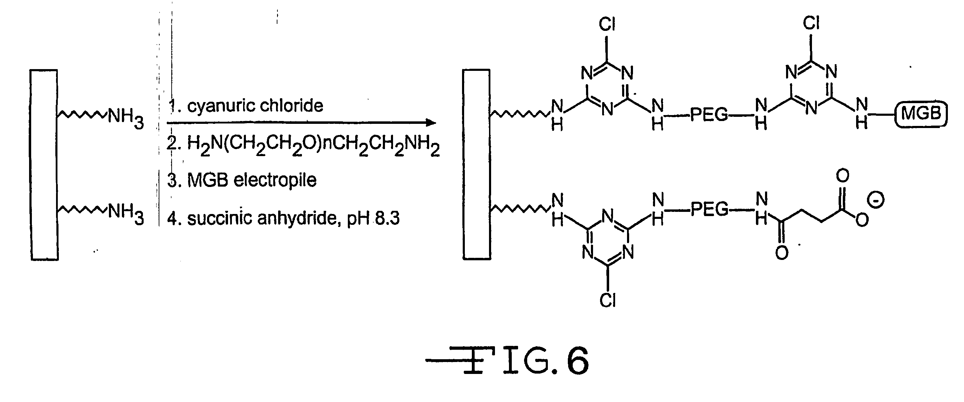

Amine modified glass and plastic supports are available as 1x3 inch microscope slides and are a common substrate for DNA microarrays. Most glass slides are made from soda lime glass (Erie Scientific, Portsmouth, NH) and modified with (gamma) aminopropylsilane groups to give a uniform density of primary amines. GAPS slides are also available with other glass types (Corning Glass), or with increased roughness (Erie). Aminopropyl coated plastic slides are also available (Nunc, Denmark) for use. Polyester films (Mylar) functionalized with amines are also available as sheets (Diagnostic Laminations Engineering, Oceanside, CA or Adhesive Research, Inc., Glen Rock, PA) and will also be examined. These substrates will either be used directly for immobilization of electrophilic MB analogs, or they will be "activated" with bifunctional linkers to give an electrophilic surface. FIG. 6 shows one possible conjugation chemistry system. Cyanuric chloride activation should be examined, as well as other aliphatic bifunctional linkers (Pierce Chemical Co.). Glass slides can be activated in anhydrous organic solvents with hydrophobic electrophiles such as cyanuric chloride to avoid hydrolysis of reagents and achieve higher densities of electrophilic modification. Thus, high loading of PEG amines (or other polyamines) may be expected. Direct coupling of fluorogenic MB electrophiles (for example, the cyanurate of FIG. 5) will be attempted first, and this (no linker) fluorogenic surface should be characterized and used as a control for fluorescence studies of the various surfaces.

Plastic slides require milder aqueous activation conditions and carbodiimide coupling or the use of activated ester linkers. PEG macromolecules containing NHS esters at each terminus are commercially available. Commercially available polycarboxylate dendrimers can possibly be coupled to amine containing supports using water soluble carbodiimide (EDC) chemistry. The hydrophilic Generation 3.5 and 4.5 PAMAM dendrimers are particularly attractive since these molecules have 64 and 128 surface carboxylates respectively. These molecules do not exhibit the dense "starburst" crowding that affects larger dendrimers. After blocking residual amines on the surface (with acetic anhydride) the carboxylates on the linker can be activated again with EDC and coupled to the hexylamine containing MB, as can be seen in FIG. 5. After thorough washing, the amount of MB covalently bound to the slide is measured by absorbance on the plate reader. The Hoechst 33258 chromophore absorbs at 356 nm, and the Bio-Tek plate reader can be set up with appropriate filters to measure this wavelength directly on microscope slides. The slides can be dried, and evaluated for future use. Stability of monolayers of fluorescent dyes on the surfaces (storage conditions) should be investigated.

The macromolecular linker structures will act as scaffolds to display the fluorogenic MB sensors to dsDNA in solution. The fluorescent properties of surface structures should be examined in the presence and absence of dsDNA. It is important that the surfaces be fully hydrated when they are evaluated for fluorogenic DNA binding properties, as H33258 derivatives are known to fluoresce when dried down on glass. Equilibration times are more rapid in a microfluidic environment, and this may be an advantage. Fluorescent background of the various linker structures will first be measured by attaching a "perfusion chamber" to the surface of the slide of interest. These are simple rubber gaskets that are adhered to the slides, and then covered with a cover slip that has an inlet port and outlet port. After the chamber is constructed, the analyte solution of interest is pipetted in and the ports can be sealed. A 200 uL chamber size will be used.

Various size DNA samples will be obtained from an outside source (Sigma Chemical Co.). The genomic DNA will likely need to be sheared to avoid tangling as it passes through the microchannels under the mild flow conditions used in the microfluidics device. Shearing of genomic DNA is easily accomplished by pulling the solution several times through an 18 gauge needle. MW standards of various lengths (40 kb, 4000 bp, 400 bp, and 40 bp) should also be considered. First, neutral buffer will be added over the fluorogenic substrate of interest, and fluorescent background (at 458 nm and 492 nm) is measured over time on the plate reader (excitation = 356 nm). Stable background measurements should be achieved when the MB coating is fully hydrated. Then, various concentrations of dsDNA (measured by A₂₆₀, 50ug/mL of dsDNA = 1 OD unit) will be introduced into the perfusion chamber, making sure to completely flush the chamber between readings. 458 nm fluorescence (characteristic of dsDNA binding) should increase in a linear fashion as DNA concentration increases. It is likely that density of MB is related to the dynamic range of measurement, and this should be considered. As described earlier, the density of MB packing is determined from the A₃₅₆ of the substrate. The microfluidics device will be handling highly concentrated DNA solutions, and, thus, a DNA concentration "threshold" for fluorescence is the goal. If DNA concentration is below the fluorescent threshold, then the isolation procedure is continued. Likewise, if fluorescent signal is above the threshold, then the required minimum DNA concentration has been achieved. Note that DNA detection using fluorogenic glass supports will be examined under physiological salt concentrations, where DNA binding by the glass should be minimal.

Concurrent with the development of fluorogenic surfaces, the parameters required for efficient immobilization and release of dsDNA from glass using small volumes of solutions is to be examined. Release of dsDNA into small volumes of buffer will be optimized by mathematical modeling. The processing steps in binding DNA to glass depend on the nature of the biological sample and there are many variations in methods. The steps described below were worked out for DNA rich sources and silica particles. Cells are first lysed by adding a 2M solution of guanidinium thiocyanate (GuSCN) buffer (pH 6.4). This chaotropic salt solution also removes histone proteins from the genomic DNA, inactivates nucleases, and drives DNA-silica complex formation. The immobilized DNA-silica is vortexed and centrifuged (this likely shears the long DNA strands), and cellular debris is washed away with more GuSCN buffer. Finally the DNA silica is dried and the purified DNA is eluted in a low salt buffer and measured by absorbance at 260 nm. Although the process is simple, these steps require piecework and human input to insure that the silica particles are homogeneous after vortexing. A microfluidics format should streamline the process as described in Table A below:

**TABLE A**

| | |
|---|---|
| 1. | lyse cells, remove histones and bind DNA to glass with high GuSCN |
| 2. | wash glass bound DNA with high GuSCN |
| 3. | release bound DNA from glass with physiological salt |
| 4. | read concentration of released DNA in chamber with fluorogenic substrate |
| 5. | release the DNA from fluorogenic substrate and formulate for use in PCR based assays |

To evaluate the DNA binding capacity of glass substrates (microscope slides), only steps 2, 3, 4 and 5 will be examined. The processing steps can be executed in perfusion chambers with glass cover slips. Mixing various size and concentrations of DNA with high GuSCN is critical for the DNA binding. The goal is to approximate the published DNA binding capacity of 300 ng between the glass surfaces of a slide and cover slip. After binding, the chamber is drained of high GuSCN and filled with physiological salt. After reaching equilibrium, the bound DNA should be free in solution and collected from the chamber. An aliquot of the concentrated DNA is measured after release, using the published fluorogenic method with H33258. Since the DNA will ultimately be used for PCR based assays, elution buffers will be consistent with this application.

The DNA binding capacity of fluorogenic MB substrates will also be examined. The same perfusion chamber system will be used to examine various DNA concentrations. The binding capacity will be examined using a variety of buffers that would be encountered in the ultimate device. Of particular interest is the released condition of dsDNA from the MB coated substrates. It is likely that bound DNA duplexes will need to be denatured to be released from the substrates, as this is the case with the methidium DNA capture beads. Dilute NaOH solutions were used in this case, and finally the denatured DNA solutions were neutralized with ammonium acetate. Alternatively, heat or very low salt can be used to denature the DNA.

The binding and release data produced in will be used as the basis for the multiphysics model that will guide the design of the microfluidics device. This model will calculate surface binding, diffusion, advection, and the resulting local concentrations of salt and DNA based on prototype geometry, solvent and solute properties, and the binding behavior characterized by the generated data. The device design with best predicted performance will become the desired prototype design. The overall performance will be compared to the predictions of the model, which will provide knowledge of the local physics in the interior of the device that cannot be experimentally obtained.

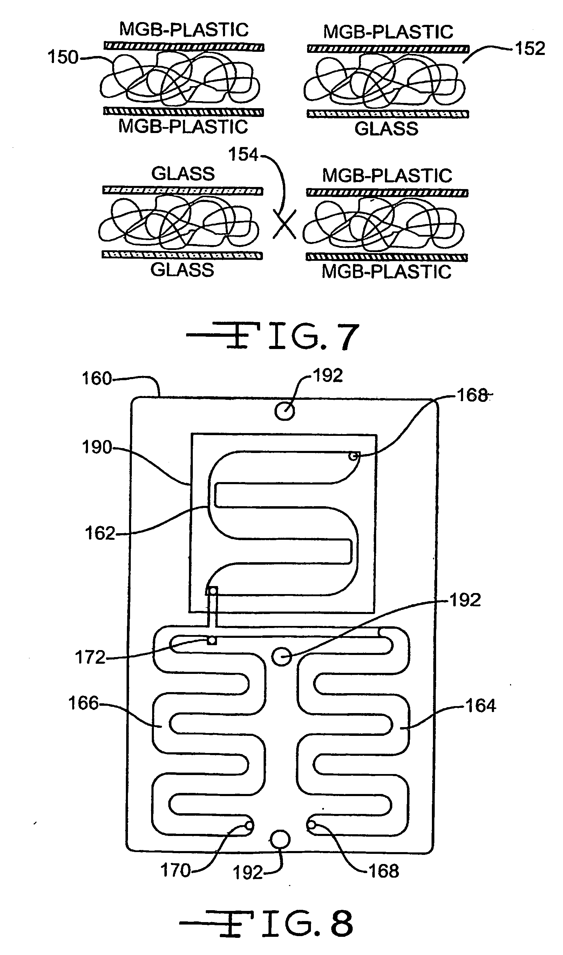

Depending on the properties of the various surfaces, several designs are feasible, as can be seen in FIG. 7. For example, it may be that a fluorogenic MB support 150 can serve dual purpose for DNA binding and measurement analogous to the use of methidium particles. Glass surfaces may have greater binding capacity, and a single chamber design 152 could combine a glass capture surface with a fluorogenic MB surface for DNA measurement. It may be that different conditions are required for DNA binding and measurement and a device 154 using two chambers would be the best design. Preliminary calculations suggest that a single chamber design with DNA binding on a glass surface and MB measurement on an opposite surface of plastic as illustrated in FIG. 8 would be able to capture and measure 84 ng of DNA even with a smooth glass surface (a rough glass surface could bind significantly more DNA).

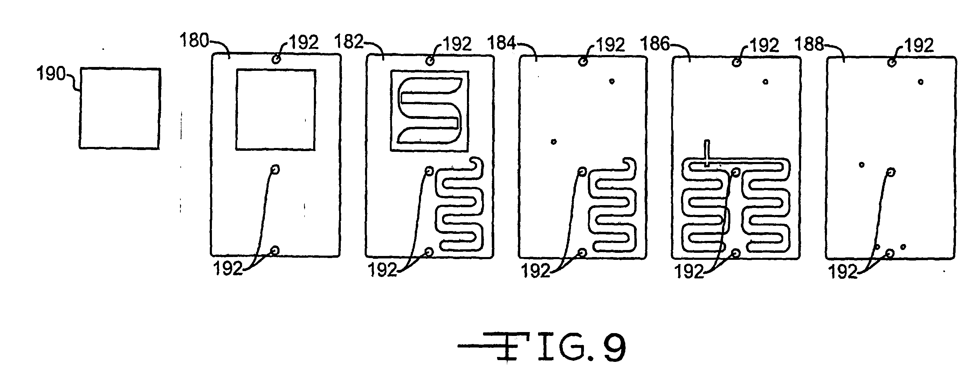

FIG. 8 shows a 62 by 40 by 3.55 nm microfluidic device 160 that contains a 5 µl DNA binding chamber 162, a 750 µl waste channel 164 and a 25 µl channel 166 to hold the released DNA at the end of the process. Fluids are inserted by pipette through an injection port 168. Fluid proceeds to waste channel 164 or released DNA channel 166 depending on whether a waste vent 168 or an outlet port 170 is open. The released DNA can be removed from card 160 by pipette from either a product vent 172 or outlet port 170. FIG. 9 shows the exploded view of all 5 laminate layers that constitute the device. Layer 180 is composed of 0.125 mm vinyl; layer 182 is a 0.025 mm adhesive layer; layer 184 is 3.175 nm PMMA; layer 186 is 0.100 mm ACA; layer 188 is 0.125 mm vinyl and layer 190 consists of a Pyrex cover slip insert which fits into layer 180. Each layer contains 3 registration holes 192 for alignment purposes. Layers 182 and 186 contain adhesive that hold the device layers together in a leakproof manner.

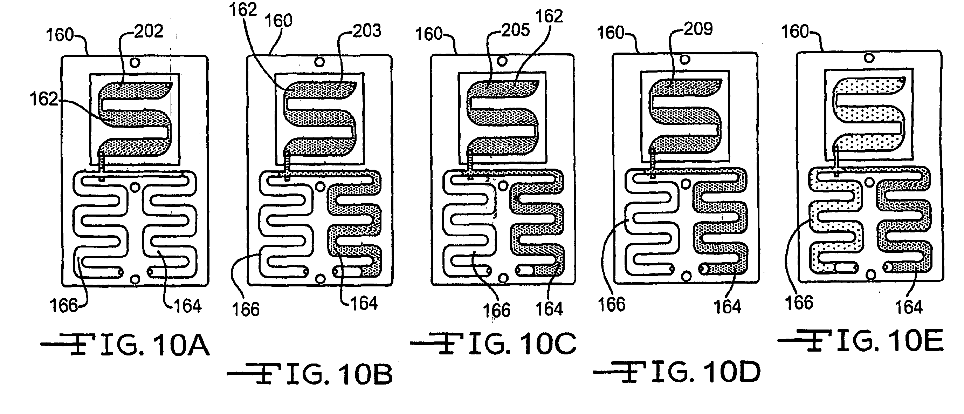

The processing steps of Table A are illustrated sequentially in FIGS. 10 A-E. FIG. 10A shows microfluidic device 160 after the injection by pipette of a mixture 202 of DNA in high salt GuSCN containing lysed sample cells. After washing with high salt GmSCN 203 flushed through binding channel 162 to waste channel 164 (FIG. 10B), chamber 162 is flushed with physiological salt 205 (FIG. 10C), UNA released from glass 190 by salt 205 attaches to MBs which luminesce such that the dsDNA concentration 209 can be read (FIG. 10D). Finally, deionized water (DI) is pipetted through binding channel 162 to densture the DNA and carry it into released DNA channel 166 for the final distribution of purified from chamber 162, as shown in FIG. 10E. At this point, product vent 132 (see FIG. 8) could be opened to retrieve the DNA. The device is small enough to easily fit on a 96-well plate reader and designed so that 6 well-center locations are within the binding channel for accurate multiple readings of the DNA concentration. At the end of the process, the immobilized fluorogenic MB groups (presumably now non-fluorescent) will be left on the solid support, and disposed of with the rest of the device in the lab waste.

### EXAMPLES

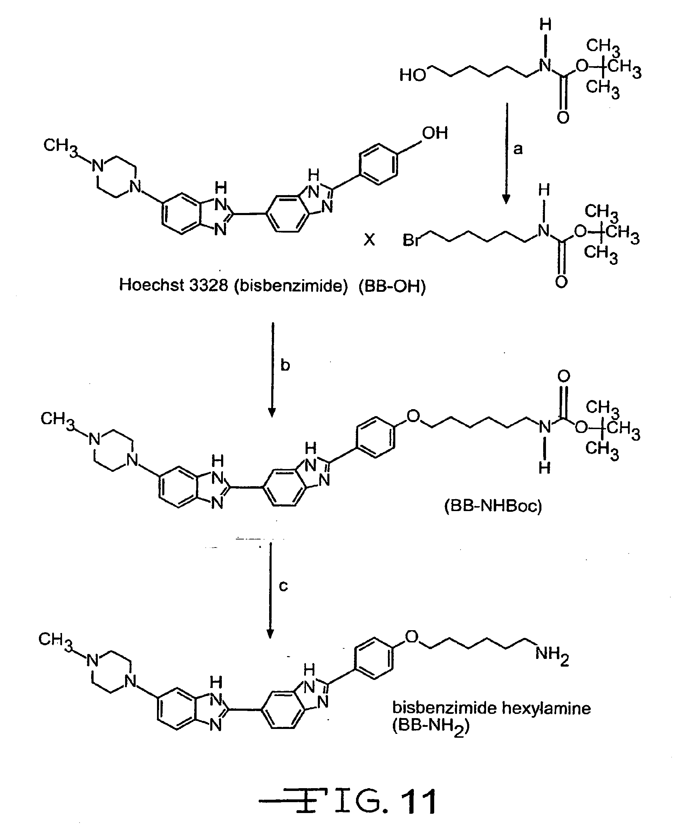

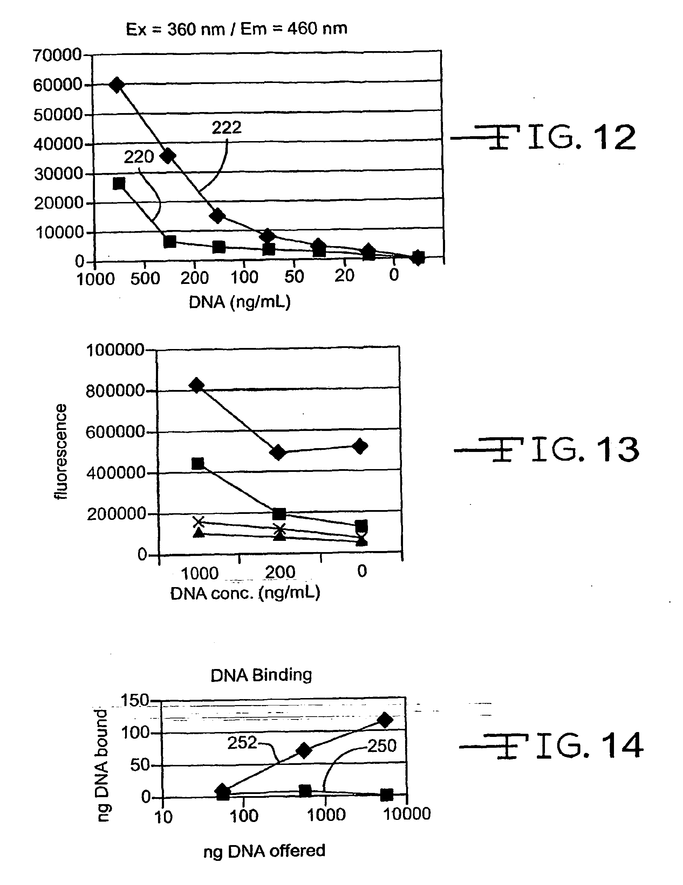

Synthesis of the fluorogenic Hoeohst dye with an attached hexylamine linked was executed. Referring now to FIG. 11, the synthesis is shown. Unless otherwise mentioned, reagents were obtained from Sigma-Adrich. Anhydrous solvents were obtained in sure-seal bottles. 7 cm long TLC strips were cut from 5x7 cm silica coated aluminum sheets (Merck), and were generally visualized by fluorescence using a hand-held long wavelength UV lamp to irradiate. Mobility of the fluorescent spot relative to the solvent front (Rf) was measured for the TLC assay. The Hoechst 33258 standard (bisbenzimide, BB-OH) used for the fluorescent DNA binding assays was obtained in a DNA Quantitation kit sold by Sigma. The kit contains bisbenzimide (10 mg/mL in water), 10x fluorescent assay buffer (10x FAB = 100mM Tris HCl, 10 mM EDTA, 2 M NaCl, pH 7.4), and a DNA standard (sheared calf thymus DNA, 1 mg/mL in 1xFAB). This kit was used as the standard for measurement of the fluorogenic measurement of DNA shown in FIG. 12.

The butyloxycarbonyl (Boc) protected aminohexanol starting material was purchased from Sigma and 500 mg was converted to the desired alkyl bromide according to a published procedure (Keller and Haner, Helv. Chim. Acta, 76 (1993) 884-892). The product was isolated by column chromatography over silica gel to yield 292 mg (45% yield) of the product as a pale yellow liquid. The separation of the desired product from other contaminants was made difficult by lack of a good TLC indicator for the alkylbromide. Staining in an iodine chamber worked with poor sensitivity. TLC (2:1 hexanes/ethylacetate): Rf of starting ROH = 0.17, Rf of RBr = 0.67.

The Hoechst 33258 dye (bisbenzimide, BB-OH) was purchased from Aldrich as a trihydrochloride salt (pentahydrate). 9.5 mg of the dye (15.2 umoles) was dissolved in 5 mL of dry DMF in a dry 15 mL round bottom flask. 36.5 mg (265 umoles) of potassium carbonate and 7.1 uL (8.5 mg, 30.4 umoles) of the Boc protected bromohexylamine. The mixture was stirred magnetically at 53 degrees for 6 hours and at room temperature for 2 days. TLC (10% methanol, 5% ethyldiisopropylamine, 85% dichloromethane) showed a mixture of blue fluorescent spots when irradiated with a long wavelength UV lamp (365 nm). The starting BB-OH (Rf = 0) was converted to the desired product (Rf = 0.3) and a higher mobility side product (Rf = 0.5). The supernatant DMF solution was decanted and the residual potassium carbonate washed with an additional 2 mL of DMF. The combined mixture was concentrated by rotary evaporation under vacuum. The residue was dissolved in 2 mL of 5% methanol, 5% ethyldiisopropylamine, 90% dichloromethane and applied to a 2x10 cm silica gel column (230-400 mesh) packed with the same solvent. After a 50 mL forerun was discarded, the higher mobility side product was collected in -75 mL. The progress of the chromatography could be followed using a hand-held UV lamp. The % methanol in the eluent was increased to 10%, and the desired product was isolated in 90 mL of solvent. Removal of solvent on the rotary evaporator gave 10.5 mg of yellow solid (theoretical yield = 9.5 mg). The product was one major blue fluorescent band by TLC with only traces of other fluorescent contaminants. The product was dissolved in 2 mL deuterochloroform for future NMR. A 1 mL portion of the CDCl₃ solution was deprotected as described below.

Trifluoroacetic acid (1 mL) from a sealed glass ampoule was added to 5 mg of BB-NHBoc in 1 mL of CDCl₃. The homogeneous solution kept in a sealed 1 dram glass vial. The deprotection was followed by TLC (35% methanol, 5% ethyldiisopropylamine, 60% dichloromethane). The starting BB-NHBoc (Rf = 0.7) was converted to a lower mobility salt (Rf = 0.5) that was slowly deprotected to a lower mobility product (Rf = 0). After 24 hours a 100 uL aliquot of the reaction mixture was converted to the free base by removing the excess TFA on the rotary evaporator, redissolving in methanol, and adding a small amount of potassium carbonate. The free base form of BB-NH2 obtained at room temperature showed a single major fluorescent band (Rf = 0.07) and a trace of fluorescent contaminants. The bulk reaction mixture was converted to the free base as described above, and dissolved in 3 mL of 2:1/methanol:chloroform). The product was not weighed (- 1.7 mg/mL), but yield was calculated by comparison with the UV absorbance of the starting bisbenzimide dye (BB-OH).

The UV-vis spectra of a 10 uM solution of BB-OH in 1 x FAB had a 340 nm absorbance maximum (0.18 units). A solution of BB-NH2 was prepared by dissolving 4.3 uL of the ∼1.7 mg/mL solution in 995.7 uL of 1xFAB. The absorbance maximum was at 342 nm (0.075 units). It was assumed that the BB-OH and BB-NH2 have the same extinction coefficient and concentration of UV-vis sample of BB-NH2 was calculated (10 uM x (0.075/0.18) = 4.4 uM). This gives a measured concentration of the ∼1.7 mg/mL solution as 0.7 mg/mL (0.98 mM). The yield of BB-NH2 (2.1 mg, 2.9 mmoles) corresponds to. 38% yield from the starting BB-OH. This assay is more accurate for determination of yield than weighing small amounts - the presence of salts and invisible (by NMR) trifluoroacetic acid could add error.

Fluorescence of the BB-OH molecule indicated at 220 in FIG. 12, changes from a weak emission at 492 nm to a strong emission at 458 nm in the presence of dsDNA. The DNA Quantitation kit from Sigma (BB-OH based) was used to generate a standard curve on a Bio-Tek FL600 fluorescent plate reader. The assay was conducted in 96-well clear bottom plates according to the supplied protocol. A BB-OH concentration of 0.1 ug/mL was used as the indicating buffer for each DNA standard (20-1000 ng/mL). Results are shown in FIG. 12.

FIG. 12 shows that BB-NH2, indicated at 222, has retained the fluorogenic DNA detection properties of the bisbenzimide dye. Modification of the phenol on the Hoechst 33258 molecule has not altered the UV-vis absorbance (λmax ∼ 340 nm) and the hexylamine modified analog gives a strong fluorescent signal (detection at 460 nm, 40 nm filter slit width) when excited at 360 nm (40 nm slit width). The hexylamine linker improves the DNA specific fluorescence, perhaps due to increased DNA affinity with the cationic primary amine. The BB-NH2 molecule has cationic amino groups at each end of the planar bisbenzimide structure, thus anchoring it in the minor groove binder of DNA. Other DNA affinity agents (like oligonucleotides) have been shown to function as fluorogenic probes in solution.

Various immobilization chemistries for preparation of fluorogenic MB surfaces were considered. 3 major components in the design of the dsDNA detecting surfaces are:
1. Indicator. fluorescent only in the presence of dsDNA; linker attaches to spacer molecules with little fluorescent background
2. Substrate. transparent in spectral regions of interest; easily coated with spacer / indicator molecules
3. Spacer. easily conjugated to the fluorogenic indicator and the substrate; allows access of the fluorogenic MB to dsDNA without steric hindrance

The BB-OH and BB-NH2 DNA indicators described in FIG. 12 have the desired DNA specific fluorescent properties and have unique functional groups that can serve as attachment points. Reaction with electrophiles such as activated esters or cyanuric chloride can occur at multiple positions in the molecules and these side reactions can decrease the desired DNA specific fluorescence. "Damaged" structures in the crescent shaped DNA binding region can alter DNA specific fluorescence. Both molecules have a cyclic tertiary alkyl amine group at one terminus that can be alkylated. The imidazole groups also present a possible site for alkylation. Various surface structures can have desirable DNA specific fluorescence, but it is advantageous to have a homogeneous and reproducible surface structure to give consistent DNA specific fluorescence. The BB-NH2 linker contains a primary hexylamine group that can be selectively reacted with electrophiles. If needed, the linker arm can be extended with a polyethylene glycol linker. Quantum Biodesign sells a Boc protected carboxylic acid that would provide a more hydrophilic and conformationally flexible linker for attachment of the BB fluor to the solid support (substrate).

Interest in plastic (mylar) substrates was dampened when it was discovered that significant background fluorescence occurs at 460 nm when irradiated with 360 nm light. Scotch™ tape (used to seal ports on the device) was also found to have significant 460 nm fluorescence. Thus, the experiment focused on glass substrates. There is a rich literature and several chemistries that exist for immobilization of oligonucleotides to 1x3 cm slides for DNA microarrays. Amine coated slides (aminopropylsilane) were obtained from Sigma as a substrate for nanoengineering of the linker and indicator molecules. Another commercially available slide chemistry (CodeLink™ from Amersham Biosciences) was also examined. These slides have an extended linker structure that terminate in activated ester (N-hydroxysuccinimide ester) groups. The CodeLink slides claim to "allow immobilization of amine terminated oligonucleotides and give hybridization without need for long PEG linkers". Since the BB-NH2 fluor could be reacted directly with this surface, it was examined first.

The immobilization of the BB-NH2 was attempted under a cover slip on the 3D-Link slides at pH 8.5. A multiwell hybridization chamber was purchased (Grace Bio-Labs) that allows up to 16 wells per slide to be examined simultaneously. After reacting for various times, the slides were rinsed free of excess BB-NH2 with buffer and capped using the same cover slip method. Final rinsing with 1xFAB took place before evaluation of fluorogenic DNA binding properties.

The properties of the BB-NH2 indicator can presumably be improved The fluorescent background at 460 nm is a key property that can be optimized if high background or poor release of dsDNA is a problem, then other surfaces will be explored using the amine coated slides. One key variable that will be explored with the CodeLink slides is the effect of capping with various amines after immobilization of the BB-NH2. This will alter the molecular properties at the interface of the BB fluorogenic molecule, the spacer and the dsDNA in solution. The nature of this interface is likely to affect the binding of fluor to dsDNA and the binding kinetics of the DNA to the solid surface. CodeLink recommends capping with 50 mM ethanolamine, and this should give a neutral surface coating. Capping with diamines or other polyamines could give a net positive charge to the slide surface and bind DNA irreversibly (no release). On the other hand, these positive charges could speed up the rate of binding or increase the local concentration of dsDNA (better signal). Capping the activated esters with lysine groups would give a zwitterionic aminoacid surface that is non-attractive to DNA, thus "passivating" the surface. Another method for introducing carboxylate groups onto the surface is to simply hydrolyze the NHS esters after immobilization of the BB-NH2. Since the immobilization will take place at high pH (8.5), keeping the slides in this buffer overnight will likely hydrolyze the available NHS esters on the slide surface. This would give a net negative charge at the interface of the slide surface and the DNA, and may affect the fluorescent signal and background. This can also be used as a method for immobilizing cationic fluorogenic indicators.

CodeLink Slides were obtained from Amersham Biosciences and used according to the following protocol. A single slide was used for examination of 16 different immobilization reactions. A 16 well silicone rubber gasket was adhered to the plate using a "96-well format" clamp system (ProPlate™ Grace Bio-labs). The square wells that are created on the slide contain a volume of up to 300 uL, and can be covered with a clear plastic adhesive sealing film (provided). The activated ester slide surface faces up and the "CodeLink" name on the slide is positioned at the top of wells 1 and 2 for orientation purposes. The BB-OH (1 mg/mL in water) and BB-NH2 (0.7 mg/mL in methanol) solutions were prepared as previously described. pH 8.5 bicarbonate (0.1 M) containing 3 mg/mL aminoethanol (50 mM) was used as the Cap Solution.

The BB-NH2 solution (100 uL) was mixed with 300 uL of methanol and diluted with 600 mL of pH 8.5 sodium bicarbonate (0.1 M). This solution (70 ug/mL) was used to prepare 7 ug/mL BB-NH2 and 0.7 ug/mL solutions in pH 8.5 bicarbonate. The solutions had a pale green fluorescence under long wavelength UV (356 nm) lamp. A 10 ug/mL BB-OH solution in pH 8.5 bicarbonate was prepared from the 1 mg/mL solution. The 16 wells on the CodeLink slide surface were treated as follows: wells 1-4, 0.7 ug/mL BB-NH2; wells 5-8, 7 ug/mL BB-NH2; wells 9-12, 70 ug/mL; wells 13-16, 10 ug/mL BB-OH. After filling the wells (0.2 mL per well) the slide module was covered with adhesive film and kept at room temperature for 18 hours (in the dark). The film was removed, the BB containing solutions were removed from each well, 0.2 mL of Cap Solution was added and the wells were re-sealed. After 7 hours, the Cap Solution was removed from each well and the gasket was removed from the slide for washing. The slide was soaked in 40 mL of 40% methanol 60% sodium bicarb (0.1 M) in a 50 mL centrifuge tube 20 min; removed and soaked in 1x FAB for 20 min. The slide was removed and analyzed on the fluorescent plate reader.

Sensitivity and accuracy of the DNA indicating surfaces was characterized. Evaluation of the BB-NH2 containing slide surfaces were examined by using the dsDNA standards previously described and a similar plate reader assay format. The BB-NH2 treated wells and BB-OH treated wells previously prepared were evaluated. The 16 well silicone gasket was reassembled on top of the treated and CodeLink slides. The residual 1x FAB buffer was removed from the slide surface by tapping on a Kimwipe (some droplets remained). Each of the 4 wells for each immobilization mixture were rehydrated with one of the following solutions: 1000 ng/mL DNA, 200 ng/mL DNA, 1xFAB, no liquid. The freshly prepared solutions were examined for 460 nm fluorescence as usual (bottom read, sensitivity = 115). After reading the wells, the DNA was removed by washing with deionized water and remeasured.

All of the BB treated slide surfaces showed increase in fluorescence at 460 nm in the presence of dsDNA as shown in FIG. 13. The immobilization reactions with highest concentration of BB-NH2 (70 ug/mL), shown at 230, showed the largest fluorescent signal. However, this high loading level also gave high fluorescent background (>50,000 counts with no DNA). The 7 ug/mL immobilization reaction, shown at 232, gave the best performance. A dose response was observed down to 200 ng/mL of DNA. The lowest level of BB-NH2 in the immobilization reaction, shown at 234, also gave a dose response down to 200 ng/mL of DNA. Although the signals were low, even the BB-OH indicator, shown at 236, gave the desired increase in 460 nm fluorescence with increased DNA concentration. After washing the DNA out of the wells with water, the wells showed no evidence of DNA specific fluorescence at 460 nm. All wells with the same level of immobilized fluor had similar fluorescence.

There is no fluorescent dye in solution in the FIG.13 data - the DNA binding assay uses the immobilized BB-NH2 or BB-OH. All surfaces show some fluorescent signal at 460 nm in the BB-NH2 immobilization region, even in the absence of DNA. When dsDNA is introduced to the indicating surface, 460 nm fluorescence increases. A control region of the slide surface (no DNA) can be used to allow background subtraction.

The immobilized BB-NH2 fluor gives good dose response in the presence of dsDNA. The fluorescent signal strength at 460 nm is about the same as the BB-NH2 response in solution, but background is higher (see FIG. 13). The major advantage of the immobilized BB-NH2 is the small volume requirement. Unlike the 200 uL well volume required for the solution phase assay, only enough DNA solution is required to fully wet the immobilized BB surface. This will allow the preparation of microfluidic devices for DNA purification.

The use of glass surfaces to purify DNA from complex biological mixtures has been shown by others, and these results have been verified using the fluorogenic BB assay. The steps required for the microfluidic device are shown below in Table B. The solution phase BB assay was used to determine DNA binding and release levels of unmodified glass.

**TABLE B**

| | |
|---|---|
| 1. | lyse cells, remove histones and bind DNA to glass with high GuSCN |
| 2. | wash glass bound DNA with high GuSCN |
| 3. | release bound DNA from glass with physiological salt |
| 4. | read concentration of released DNA in chamber with fluorogenic substrate |
| 5. | release the DNA from fluorogenic substrate and formulate for use in PCR based assays |

Guanidinium thiocynate (10M) was examined in the fluorogenic DNA binding assay and found to give fluorescent background at 460 nm with high concentrations. Another common chaotrope for DNA binding to glass is sodium iodide (12M). It had low background, but is more expensive than sodium perchlorate, and is more difficult to handle (solution discolor). Therefore, sodium perchlorate (6M) was chosen for development. Solutions of 50, 500 or 5000 ng of DNA in 0.1 mL were prepared in 6M sodium perchlorate (pH 8 Tris). These 0.1 mL volumes were transferred to polystyrene Petrie dishes and 22x22 mm cover slips (glass or plastic) were placed over them. After 1.5 hours at room temperature, the slips were carefully tilted up and removed from the residual liquid. The slips were placed on Kimwipes (DNA side down) to remove any residual droplets. Each slip was transferred to a 0.1 mL volume of 1 x fluorescence assay buffer in a Petrie dish (1x FAB: 10 mM Tris HCl, 1 mM EDTA, 200 mM NaCl, pH 7.4). After 4 hours at room temperature, the slips were removed and the DNA side was rinsed with 0.15 mL of 1 x FAB. The combined buffer from each slide was brought to 0.27 mL with 1x FAB and 30 uL of 1 ug/mL bisbenzimide (BB-OH) solution (in 1x FAB) was added. 200 microliter volumes from each slip were transferred to a microwell (clear bottom) and the samples were read on a Biotek FL-600 plate reader. The fluorescence was read at 460 nm and DNA concentration was determined from a standard curve.

Referring now to FIG. 14, the efficiency of DNA capture and release from cover slips is shown. The plastic cover slips show no DNA binding at 250. The efficiency of DNA capture and release from glass cover slips, shown at 252, is greatest for low amounts of DNA. At 50 ng of DNA per cover slip, 7.2 ng was recovered (14% efficiency). At 500 ng of DNA per cover slip, 68 ng was recovered (14% efficiency). At 5000 ng of DNA per cover slip, 113 ng of DNA was recovered (2% efficiency). These results show that the glass cover slips are suitable surfaces for microfluidic DNA purification, and at least 68 ng of DNA can be efficiently recovered from a 500 ng sample.

Prototype cards have been optimized for hand-pipette addition of solutions, and a flow rate of 100 uL per minute can be achieved with a trained hand and Rainin 200 uL Pipettman. Slower flow rates (important to minimize bubble formation) are more difficult to achieve, but a 200 uL pipettor was successfully used to fill the DNA binding channel (20 uL) without introducing bubbles.

The cards discussed previously were successful with some minor variations. The glass lined channels wetted well with low flow rates, and fluid could be removed by flowing air through the channels. The DNA harvest well was designed to collect all of the purified DNA for analysis using a fluorescent plate reader. With the immobilized BB surfaces, the DNA harvest well can be designed to provide a low profile channel which can be easily configured to distribute the DNA to other channels after fluorescent analysis. Since the fluorogenic BB dye is immobilized, the purified DNA is not contaminated with indicating fluor when transferred to downstream DNA analysis chambers.

## Claims

1. A device (160), comprising:
(a) an inlet port (168);
(b) an outlet port (170); and
(c) a single binding channel (162) in liquid communication with the inlet port and outlet port, wherein said binding channel has a rectangular cross-section, and a top wall, a bottom wall, and two side walls and wherein one or two of said walls is an unmodified smooth glass surface effective for binding nucleic acids and the other walls are plastic.

2. The device of Claim 1, wherein the nucleic acids are double stranded DNAs or single stranded DNAs.

3. The device of Claim 1, wherein the nucleic acids are RNAs.

4. The device of Claim 1 further comprising means for quantitating the nucleic acids.

5. A method for capturing nucleic acids from chaotropic salt solutions, comprising passing a chaotropic salt solution containing nucleic acids through a device (160) according to claim 1, whereby the solution containing nucleic acids is contacted with the unmodified smooth glass surface to provide immobilized nucleic acids.

6. The method of Claim 5, wherein the immobilized nucleic acids are double stranded DNAs or single stranded DNAs.

7. The method of Claim 5, wherein the immobilized nucleic acids are RNAs.

8. The method of Claim 5 further comprising washing the immobilized nucleic acids.

9. The method of Claims 5 or 8 further comprising releasing the immobilized nucleic acids from the glass surface to provide released nucleic acids.

10. The method of Claim 9 further comprising quantitating the released nucleic acids.

## Patentansprüche

1. Vorrichtung (160) umfassend:
(a) eine Eintrittsöffnung (168);
(b) eine Austrittsöffnung (170); und
(c) ein einzelner Verbindungskanal (162) in Flüssigkeits-Kommunikation mit der Eintrittsöffnung und Austrittsöffnung, wobei der Verbindungskanal einen rechteckigen Querschnitt hat, und eine obere Wand, eine untere Wand, und zwei Seitenwände und wobei eine oder zwei der Wände eine unbehandelte glatte Glasfläche effektiv zum Binden von Nukleinsäuren ist und die anderen Wände Kunststoff sind.

2. Vorrichtung nach Anspruch 1, wobei die Nukleinsäuren doppelsträngige DNSn oder einzelsträngige DNSn sind.

3. Vorrichtung nach Anspruch 1, wobei die Nukleinsäuren RNSn sind.

4. Vorrichtung aus Anspruch 1, weiterhin Mittel zum Quantifizieren der Nukleinsäuren umfassend.

5. Verfahren zum Einfangen von Nukleinsäuren aus chaotopen Salzlösungen, welches ein Durchleiten einer Nukleinsäuren enthaltenen chaotopen Salzlösung durch eine Vorrichtung (160) nach Anspruch 1 umfaßt, wobei die Nukleinsäuren enthaltene Lösung mit der unbehandelten glatten Glasfläche in Kontakt gebracht wird, um immobilisierte Nukleinsäuren bereit zu stellen.

6. Verfahren nach Anspruch 5, wobei die immobilisierten Nukleinsäuren doppelsträngige DNSn oder einzelsträngige DNSn sind.

7. Verfahren nach Anspruch 5, wobei die immobilisierten Nukleinsäuren RNSn sind.

8. Verfahren nach Anspruch 5, weiterhin Waschen der immobilisierten Nukleinsäuren umfassend.

9. Verfahren nach Ansprüchen 5 oder 8, weiterhin Freisetzen der immobilisierten Nukleinsäuren von der Glasfläche umfassend, um freigesetzte Nukleinsäuren bereit zu stellen.

10. Verfahren nach Anspruch 9, weiterhin Quantifizieren der freigesetzten Nukleinsäuren umfassend.

## Revendications

1. Dispositif (160), comprenant :
(a) un orifice d'admission (168) ;
(b) un orifice de sortie (170) ; et
(c) un canal de liaison unique (162) en communication par liquide avec l'orifice d'admission et l'orifice de sortie, dans lequel ledit canal de liaison a une section transversale rectangulaire, et une paroi supérieure, une paroi inférieure, et deux parois latérales, et dans lequel une ou deux desdites parois consistent en une surface de verre lisse non modifiée efficace pour la liaison d'acides nucléiques et les autres parois sont constituées d'une matière plastique.

2. Dispositif suivant la revendication 1, dans lequel les acides nucléiques sont des ADN bicaténaires ou des ADN monocaténaires.

3. Dispositif suivant la revendication 1, dans lequel les acides nucléiques sont des ARN.

4. Dispositif suivant la revendication 1, comprenant en outre un moyen pour quantifier les acides nucléiques.

5. Procédé pour capturer des acides nucléiques à partir de solutions de sels chaotropes, comprenant le passage d'une solution de sels chaotropes contenant des acides nucléiques à travers un dispositif (160) suivant la revendication 1, la solution contenant les acides nucléiques étant ainsi mise en contact avec la surface de verre lisse non modifiée pour fournir des acides nucléiques immobilisés.

6. Procédé suivant la revendication 5, dans lequel les acides nucléiques immobilisés sont des ADN bicaténaires ou des ADN monocaténaires.

7. Procédé suivant la revendication 5, dans lequel les acides nucléiques immobilisés sont des ARN.

8. Procédé suivant la revendication 5, comprenant en outre le lavage des acides nucléiques immobilisés.

9. Procédé suivant la revendication 5 ou 8, comprenant en outre la libération des acides nucléiques immobilisés de la surface de verre pour fournir des acides nucléiques libérés.

10. Procédé suivant la revendication 9, comprenant en outre la quantification des acides nucléiques libérés.
